Europäisches Patentamt

European Patent Office

Office européen des brevets

(19)

(11)  **EP 1 637 129 A1**

(12)  **EUROPEAN PATENT APPLICATION**

(43) Date of publication:
   **22.03.2006   Bulletin 2006/12**

(51) Int Cl.:
   **A61K 9/22** (2006.01)      **A61K 31/517** (2006.01)

(21) Application number: **05011338.0**

(22) Date of filing: **25.05.2005**

(84) Designated Contracting States:
   **AT BE BG CH CY CZ DE DK EE ES FI FR GB GR
   HU IE IS IT LI LT LU MC NL PL PT RO SE SI SK TR**
   Designated Extension States:
   **AL BA HR LV MK YU**

(30) Priority: **17.09.2004   US 943725
         06.05.2005   US 123723**

(71) Applicant: **Cimex Pharma AG
   4102 Benningen/Basel (CH)**

(72) Inventors:
   • **Scheer, Mathias
    79664 Wehr (DE)**

   • **Ray, Helene
    68300 St. Louis (FR)**
   • **Fischer, Marc
    4106 Therwil (CH)**
   • **Kramer, Dirk
    79276 Reute (DE)**

(74) Representative: **Symons, Rupert Jonathan et al
   HLBBshaw
   10th Floor
   1 Hagley Road
   Birmingham B16 8TG (GB)**

(54)   **Composition comprising alfuzosin**

(57)   A monolithic composition includes alfuzosin in a polymeric matrix adapted to release 13-33% of the alfuzosin within 2 hours, 40-60% of the alfuzosin within 7 hours, and greater than 80% of the alfuzosin within 20 hours of administration. A unit dosage form includes: a heterogeneous mixture of alfuzosin hydrochloride anhydrate, lactose monohydrate, hydroxypropylmethylcellulose, polyvinylpyrrolidone and magnesium stearate, wherein the heterogeneous mixture is heterogeneously distributed throughout the unit dosage form. A manufacturing process includes: mixing a hydrophilic polymer and alfuzosin to provide a blend; granulating the blend to provide granules; drying the granules on a dryer to provide dried granules; sizing the dried granules to provide sized granules; mixing the sized granules with a lubricant to obtain a mixture; and compressing the mixture to obtain a tablet. A method of treating benign prostatic hyperplasia, includes administering to a patient the composition or unit dosage form once a day.

## Fig. 1

EP 1 637 129 A1

**Description**

This invention relates to alfuzosin, and, in particular, a tablet with controlled release of alfuzosin hydrochloride and to methods for manufacturing and administering the same.

Alfuzosin is a quinazolin derivative for oral administration (in the form of the hydrochloride) that selectively blocks postsynaptic alpha-1-receptors. In vitro studies have confirmed selectivity of the substance on alpha-1-receptors in the trigone of the urine bladder, urethra and prostate, but also to the sympathomimetic nerve impulse, which by stimulating the post-synaptic alpha receptors increases the tension of the smooth muscles of the lower urinary tract thus contributing to the resistance to outflow of urine. Clinical evidence of uroselectivity has been demonstrated. In men, alfuzosin improves voiding parameters and facilitates bladder emptying.

After administration of an immediate-release tablet containing alfuzosin hydrochloride, the active agent is rapidly and well absorbed. Bioavailability is 64% (45 - 90%). Peak plasma concentrations are reached within 1 hour after administration. The kinetic profile is characterized by large (sevenfold) inter-individual fluctuations in plasma concentrations. Terminal elimination half-life is approximately 5 h (1 - 10 h).

Thus, alfuzosin has a relatively short half-life, and is more intensely absorbed at the duodenum-jejunum level than in subsequent portions of the gastrointestinal tract. Consequently, for an optimum effect, the administration of alfuzosin hydrochloride as conventional tablets (with rapid disintegration and dissolution) must be carried out several times a day. Therapy with immediate release alfuzosin tablets typically requires a daily dose of 3 tablets containing 2.5 mg alfuzosin hydrochloride. For these reasons, alfuzosin hydrochloride is a candidate for the production of a pharmaceutical preparation with controlled release in the proximal upper parts of the tract (duodenum and jejunum).

U.S. Patent No. 6,149,940 discloses such a pharmaceutical preparation, wherein alfuzosin hydrochloride ((R, S)-N-(3-(4-amino-6,7-dimethoxy-2-quinazolinyl) methylamino) propyl) tetrahydro-2-furancarboxamide hydrochloride) is provided in a multilayered tablet further comprising a hydrophilic swelling agent, such as crosslinked polyvinylpyrrolidone (also known as povidone or PVP), hydroxypropylcellulose or hydroxypropylmethylcellulose (also known as hypromellose or HPMC) having a molecular weight from 1,000 to 100,000, crosslinked sodium carboxymethylcellulose, carboxymethyl starch or its salts, or divinylbenzene/potassium methyacrylate copolymer. The swelling agent swells upon contact with gastric juices, such that the pharmaceutical preparation increases considerably in volume, which slows the passage of the pharmaceutical preparation through the stomach. In this way, most of the alfuzosin hydrochloride contained may be absorbed in a controlled manner in that portion of the gastrointestinal tract that has the highest capacity for absorption.

XATRAL LP (UROXATRAL in the U.S.) is a three-layered tablet prepared in accordance with the teachings of US 6,149,940, wherein the active alfuzosin hydrochloride layer is sandwiched between two external swelling layers containing HPMC. These 10 mg sustained-release tablets can be administered once a day to achieve clinically relevant tissue concentrations. Moreover, the bioavailability of the 10 mg sustained-release tablets is 104% when compared to 2.5 mg immediate-release tablets. Peak plasma concentrations occur approximately 6 - 9 h after oral administration of the sustained-release formulation. The elimination half-life is approximately 9.1 h after oral doses.

The pharmacokinetic profile of the sustained-release formulation permits the higher initial daily dose. Therapeutically effective alfuzosin concentrations can be reached rapidly without excessive plasma levels. More uniform plasma levels are provided with minimal peak-to-trough fluctuation. This improved absorption profile minimizes the risk for undesirable effects.

U.S. Patent Application published under No. 2004/0115259 A1 (an English-language counterpart of WO 02/062321) discloses a floating alfuzosin tablet and a method for producing the tablet. The tablet is prepared by mixing alfuzosin with a carrier selected from the group consisting of cellulose derivatives, povidone derivatives and polyvinyl acetate derivatives, and compressing the mixture with a force effective to provide a homogeneous monolithic tablet that floats in the gastric medium. Floating is achieved by providing a tablet density lower than the density of gastric fluids.

US 2004/0115259 purports to be an improvement over FR 2,784,583 (CA 2,347,105 is an English-language counterpart), which also teaches the alleged advantages of floating alfuzosin tablets for sustained release of alfuzosin. In the case of FR 2784583, the tablets are multilayered and include, as a flotation agent, an effervescent component that generates carbon dioxide when contacted with gastric fluids. US 2004/045259 faults the complexity and instability of tablets according to FR 2784583.

Despite the foregoing developments, it is desired to provide alternative means for prolonged release of alfuzosin. It is further desired to provide a prolonged release formulation which stabilizes alfuzosin hydrochloride anhydrate. It is still further desired to provide a monolithic tablet for prolonged release of alfuzosin, which is non-floating. It is still further desired to provide a monolithic tablet for prolonged release of alfuzosin, which is bioequivalent with UROXATRAL, XATRAL LP 10 mg and/or XATRAL OD. It is also desired to provide a process for producing sustained-release tablets containing alfuzosin, which is less complex than the process employed to provide multilayered alfuzosin tablets.

All references cited herein are incorporated herein by reference in their entireties.

Accordingly, the invention provides a composition comprising:

a polymeric matrix; and
alfuzosin in the polymeric matrix,

wherein: (a) the composition is monolithic in form, (b) the composition does not float in gastric fluids, and (c) the polymeric matrix is adapted to release 13-33% of the alfuzosin within 2 hours of administration, 40-60% of the alfuzosin within 7 hours of administration and greater than 80% of the alfuzosin within 20 hours of administration.

In certain embodiments, the alfuzosin is an anhydrous salt.

In certain embodiments, the alfuzosin consists essentially of alfuzosin hydrochloride anhydrate.

In certain embodiments, the composition is a tablet free of layers.

In certain embodiments, the alfuzosin is homogeneously distributed throughout the composition.

In certain embodiments, the polymeric matrix is homogeneously distributed throughout the composition.

In certain embodiments, the polymeric matrix predominantly comprises a hydrophilic polymer adapted to gel or swell upon contact with gastrointestinal fluids.

In certain embodiments, the hydrophilic polymer is hydroxypropylmethylcellulose.

In certain embodiments, the composition further comprises lactose monohydrate, polyvinylpyrrolidone and magnesium stearate.

In certain embodiments, the composition comprises 1-30 mg alfuzosin hydrochloride anhydrate, 2-100 mg lactose monohydrate, 20-800 mg hydroxypropylmethylcellulose, 2-100 mg polyvinylpyrrolidone and 0.1-25 mg magnesium stearate.

In certain embodiments, the composition comprises about 10 mg alfuzosin hydrochloride anhydrate, about 7.8 mg lactose monohydrate, about 255 mg hydroxypropylmethylcellulose, about 24mg polyvinylpyrrolidone and about 3.0 mg magnesium stearate.

In certain embodiments, the composition is adapted to induce a peak plasma concentration of alfuzosin about 6 hours to about 9 hours after oral administration.

In certain embodiments, an elimination half-life of the composition is about 9 hours after oral administration.

In certain embodiments, the composition is produced by a dry, aqueous or organic granulation process.

Also provided is a composition comprising:

a polymeric matrix; and
alfuzosin in the polymeric matrix,

wherein: (a) the composition is monolithic in Form, (b) a 300 mg tablet of the composition having a hardness of 100 N sinks within one minute of being placed in one liter of a 0.01 N HCl solution in a USP Type II apparatus with a paddle speed of 100 rpm and a temperature of 37±2°C, and remains below the surface for at least 20 minutes, and (c) the polymeric matrix is adapted to release 13-33% of the alfuzosin within 2 hours of administration, 40-60% of the alfuzosin within 7 hours of administration and greater than 80% of the alfuzosin within 20 hours of administration.

In certain embodiments of this composition, the alfuzosin is an anhydrous salt.

Further provided is a unit dosage form comprising a heterogeneous mixture of alfuzosin hydrochloride anhydrate, lactose monohydrate, hydroxypropylmethylcellulose, polyvinylpyrrolidone and magnesium stearate, wherein the heterogeneous mixture is heterogeneously distributed throughout the unit dosage form, and the unit dosage form does not float in gastric fluids.

In certain embodiments, the unit dosage form comprises 1-30 mg alfuzosin hydrochloride anhydrate, 2-100 mg lactose monohydrate, 20-800 mg hydroxypropylmethylcellulose, 2-100 mg polyvinylpyrrolidone and 0.1-25 mg magnesium stearate.

In certain embodiments, the unit dosage form comprises about 10 mg alfuzosin hydrochloride anhydrate, about 7.8 mg lactose monohydrate, about 255 mg hydroxypropylmethylcellulose, about 24mg polyvinylpyrrolidone and about 3.0 mg magnesium stearate.

In certain embodiments, the unit dosage form is adapted to release 13-33% of the alfuzosin within 2 hours of administration, 40-60% of the alfuzosin within 7 hours of administration and greater than 80% of the alfuzosin within 20 hours of administration.

In certain embodiments, the unit dosage form is a monolithic tablet.

In certain embodiments, the unit dosage form has a pharmaceutically inactive external coating.

In certain embodiments, the unit dosage form is substantially free of polymorphs of alfuzosin other than alfuzosin hydrochloride anhydrate.

Still further provided is a process for preparing the composition and/or unit dosage form of the invention, said process comprising:

mixing a hydrophilic polymer and alfuzosin to provide a blend;

granulating the blend to provide granules;

drying the granules on a dryer to provide dried granules;

sizing the dried granules to provide sized granules;

mixing the sized granules with a lubricant to obtain a mixture; and

compressing the mixture to obtain a tablet.

In certain embodiments, the hydrophilic polymer is hydroxypropylmethylcellulose and the alfuzosin is alfuzosin hydrochloride anhydrate.

In certain embodiments, the granulating comprises dry granulating the blend or adding a granulation liquid to the blend and wet granulating the blend.

In certain embodiments, the blend comprises hydroxypropylmethylcellulose, alfuzosin hydrochloride anhydrate, polyvinylpyrrolidone and lactose monohydrate, the granulating comprises dry granulating or wet granulating with a granulation liquid comprising at least one of alcohol and water and optionally polyvinylpyrrolidone, and the lubricant comprises magnesium stearate.

In certain embodiments, the tablet contains 1-30 mg alfuzosin hydrochloride anhydrate, 2-100 mg lactose monohydrate, 20-800 mg hydroxypropylmethylcellulose, 2-100 mg polyvinylpyrrolidone and 0.1-25 mg magnesium stearate.

In certain embodiments, the tablet contains about 10 mg alfuzosin hydrochloride anhydrate, about 7.8 mg lactose monohydrate, about 255 mg hydroxypropylmethylcellulose, about 24mg polyvinylpyrrolidone and about 3.0 mg magnesium stearate.

Still further provided is a method of treating benign prostatic hyperplasia, said method comprising administering to a patient a composition or unit dosage form of the invention once a day, wherein the composition does not float in the stomach of the patient.

In this specification, the terms 'non-floating', 'sinking' and like or similar terms are to be understood to relate to a material or substance which is able to satisfy either or both of the following tests:

1. a density greater than gastric fluids; and/or

2. when formed into a 300 mg tablet having a hardness of 100 N it sinks within one minute of being placed in one liter of a 0.01 N HCl solution in a USP Type II apparatus with a paddle speed of 100 rpm and a temperature of $37 \pm 2°C$, and remains below the surface for at least 20 minutes,

The invention will be described in conjunction with the following drawings in which like reference numerals designate like elements and wherein:

Fig. 1 is a graph of dissolution rates of a reference tablet and the tablets of Examples 1-4;

Fig. 2 is a graph of dissolution rates ofa reference tablet and the tablets of Examples 5-8;

Fig. 3 is a graph of dissolution rates of a reference tablet and the tablets of Examples 9-12;

Fig. 4 is a graph of dissolution rates of a reference tablet and the tablets of Examples 13-15; and

Fig. 5 is a graph of dissolution rates of a reference tablet and the tablets of Examples 19-20.

The invention provides a monolithic composition containing a mixture of an active agent and a retarding agent. The composition is preferably provided in the form of a tablet.

The term "monolithic" as used in expressions such as "monolithic tablet" is intended to denote that the tablet (or other object) is not layered. The term "monolithic" is not intended to require that the tablet be formed from a single material or comprise a completely homogenous mixture.

The active agent is preferably alfuzosin or a salt thereof, more preferably an anhydrous salt, and is most preferably alfuzosin hydrochloride anhydrate. The amount of active agent in the composition can be determined with routine experimentation using the present disclosure as a guide. When the active agent is alfuzosin hydrochloride, each dosage unit of the composition preferably contains from 1 mg to 250 mg, preferably 1 mg to 30 mg, most preferably about 10 mg (i.e., 10 mg $\pm$ 1 mg) alfuzosin hydrochloride.

Alfuzosin hydrochloride used for the manufacture of the inventive composition is freely soluble in water. To ensure that the active agent is gradually released, a highly viscous matrix is provided by the retarding agent. Preferably, the alfuzosin hydrochloride is released at rates substantially identical to the release rates of UROXATRAL, XATRAL LP 10 mg and/or XATRAL OD. In certain embodiments, the *in vitro* release rate profile is as follows: 2 h: 13-33 % released; 7 h: 40-60 % released; and 20 h: $\geq$ 80% released. In certain embodiments, the composition is adapted to induce a peak plasma concentration of alfuzosin about 6 hours to about 9 hours after oral administration (to a patient) and/or the elimination half-life of the composition is about 9 hours (i.e., 9 hours $\pm$1 hour) after oral administration.

The retarding agent is a hydrophilic polymer that gels or swells upon contact with gastrointestinal fluids, such that passage of the formulation from the stomach (duodenum, jejunum, etc.) is delayed. In addition, the retarding agent provides a matrix from which the active agent cannot readily escape. Upon contact with water, alfuzosin hydrochloride dissolves and diffuses into the polymeric matrix. Thus, the release kinetic of the alfuzosin hydrochloride is governed by the relative magnitude of the rate of polymer swelling and erosion, which starts at the tablet surface and expands into the interior of the tablet with time.

Unlike the tablets of US 2004/0115259 and FR 2784583 discussed above, compositions of the present invention do not float. A lack of flotation in accordance with the present invention is defined according to the following test. A composition is deemed not to float if a 300 mg tablet of the composition having a hardness of 100 N sinks below the surface within one minute of being placed in one liter of a model gastric medium (0.01 N HCl) in a USP Type II apparatus with a paddle speed of 100 rpm and a medium temperature of $37 \pm 2°C$, and remains below the surface for at least 20 minutes. Preferably, the composition never floats.

Contrary to the teachings of US 2004/0115259 and FR 2784583, we have discovered that flotation is not an advantageous property of alfuzosin compositions. Release profiles of such compositions depend on complete contact with gastric fluids. Undesirably high variability of release rates result from incomplete and inconsistent contact. A floating tablet is critical in this respect because the entire surface of the tablet is not completely or consistently in contact with gastric fluids. In addition, the release rale will be affected by different stomach filling levels (empty versus filled) and contents (food versus drinks).

It is know that different polymorphs of a pharmaceutically active ingredient may vary in the bioavailability and/or absorption. For this reason, it is preferred to avoid any transformation in the pharmaceutical formulation. Preferred compositions of the invention stabilize the anhydrous polymorph and prevent the formation of the dihydrate polymorph of alfuzosin.

Suitable hydrophilic polymers for use as the retarding agent are biocompatible. They are slowly soluble and/or slowly gelable and/or swell rapidly or at a different rate in aqueous liquids and then may optionally be broken down. Preferred polymers include hydroxymethylcellulose, hydroxyethylcellulose, hydroxypropylmethylcellulose having a molecular weight of from 1000 to 4,000,000, hydroxypropylcellulose having a molecular weight of from 2000 to 2,000,000, carboxyvinyl polymers, chitosans, mannans, galactomannans, xanthans, carrageenans, amylose, alginic acid, its salts and its derivatives, pectins, acrylates, methacrylates, acrylic/methacrylic copolymers, polyanhydrides, polyamino acids, poly(methyl vinyl ether/maleic anhydride) polymers, polyvinyl alcohols, glucans, scleroglucans, carboxymethylcellulose and its derivatives, ethylcellulose, methylcellulose and, in general, hydrophilic cellulose derivatives.

HPMC is the most preferred retarding agent, as it is pharmaceutically acceptable and non-ionic, such that no interactions between the polymer and other constituents are to be expected. HPMC provides a hydrophilic matrix in which the active agent is distributed uniformly, and is released over a sustained period of time.

The various HPMC quality grades commercially available differ in their molecular weight. The molecular weight of the polymer used and its concentration in the tablet are of particular importance for the release of the drug substance. Adjustment of these parameters to achieve desired results is possible with routine experimentation using the present disclosure as a guide. A higher molecular weight leads to an increase in gel strength and increased viscosity, reducing the release of the drug substance due to a greater barrier to diffusion and slower erosion of the tablet. Increasing the polymer concentration in the preparation, i.e., the ratio HPMC/drug substance, results in an increase of gel viscosity on the surface of the tablets. This delays the release of drug substance from the gel layer. The concentration effect is, however, of limited relevance for HPMC grades of high molecular weight.

The content of hydrophilic polymers may range from 5 to 90% relative to the total weight of the composition, but preferably from 80 to 90% and more preferably about 85%.

The composition can also include additives additional to the active agent and the retarding agent.

In certain embodiments, the composition can include wetting agents that are capable of facilitating interaction between the components of the composition and the biological fluids with which the composition comes into contact. Suitable wetting agents include but are not limited to anionic, cationic and nonionic surfactants. More specific non-limiting examples include sodium lauryl sulphate, sodium ricinoleate, sodium tetradecyl sulphate, sodium dioctyl sulphosuccinate, cetomagrogol, poloxamer, glyceryl monostearate, polysorbates, sorbitan monolaurate, lecithins or any other pharmaceutically acceptable surfactant.

In addition, other hydration-modifying additives may be used, such as, e.g., hydrophilic diluents such as mannitol, lactose, starches of various origins, sorbitol, xylitol, microcrystalline cellulose and/or substances which, in general, promote the penetration of water or of aqueous fluids into the pharmaceutical preparation, hydrophobic diluents such as glyceryl monostearate, palmitates, hydrogenated or unhydrogenated plant oils such as hydrogenated castor oil, waxes, mono-, di- or trisubstituted glycerides, for slowing down the penetration of water or of aqueous fluids into the pharmaceutical preparation.

The preparation of the composition, particularly the process of tableting, may introduce into the composition: lubricants such as magnesium stearate, stearic acid, glyceryl monostearate; polyoxyethylene glycols having a molecular weight of from 400 to 7,000,000; hydrogenated castor oil; glyceryl behenate; mono-, di- or trisubstituted glycerides; flow agents such as colloidal silica or any other silica; binders (e.g., povidone); buffers; absorbing agents; and other pharmaceutically acceptable additives.

In a particularly preferred embodiment, the composition of the invention comprises a tablet containing alfuzosin hydrochloride as the active ingredient, HPMC as the retarding agent, povidone as a binder, lactose monohydrate as a filler and magnesium stearate as a lubricant. Preferred ranges of these ingredients are listed in Table 1.

Table 1. Preferred Composition of Unit Dose (e.g., Tablets)

| Component | Amount per Unit (mg) | Preferred Range per Unit (mg) |
|---|---|---|
| Alfuzosin Hydrochloride | 10.00 | 1-30 mg |
| Lactose Monohydrate | 7.80 | 2-100 mg |
| Hydroxypropylmethylcellulose | 255.00 | 20-800 |
| Povidone K25 | 24.00 | 2-100 |
| Purified Water | 24.00 | 0-500 |
| Magnesium stearate | 3.00 | 0.1-25 |

During development of the tablets of the invention, various types of manufacturing processes were investigated. First attempts were made to manufacture the tablets by direct compression after blending the drug substance with suitable excipients. The results revealed that direct compression is most practical with a formulation containing 10 to 50% HPMC. Above this percentage, the flowability of the dry mix decreases, resulting in unacceptable weight variation. Since higher concentrations of HPMC are preferred, direct compression is not the preferred means for tableting.

Granulation is preferred prior to the tableting to obtain a formulation of consistent quality containing a higher amount of HPMC and good flowing properties. Granulation tends to produce tablets having better friability than tablets produced by direct compression. Perhaps more importantly, granulation most readily provides non-floating compositions of the invention. Wet granulation, fluid bed granulation and dry compaction/granulation are all suitable granulation techniques.

Thus, a preferred tableting process comprises: mixing HPMC, alfuzosin hydrochloride, povidone and lactose monohydrate in a high shear mixer to form a blend; granulating the blend (preferably by adding purified water or alcohol as granulation liquid); drying the granules on a dryer; sizing the dried granules; mixing the sized granules with magnesium stearate to obtain a mixture; and compressing the mixture (e.g., on an rotary press) to obtain tablets (e.g., flat and plain tablets having a diameter of about 8.0 mm). The blend shows no sticking, capping or insufficient hardness. The achievable hardness and friability arc acceptable using this method.

Alcohol is the most preferred granulation liquid (or solvent for the binder solution). However, alcohol and water/alcohol solvent blends are also suitable for use in the invention. The binder (preferably povidone) is preferably added to the dry mix. However, a solution of the binder in the granulation liquid is also suitable for use in the invention.

The granules obtained from the wet granulation are dried until the desired loss on drying value (e.g., about 1% to about 5%) is achieved. Preferred drying conditions are a drying temperature of about 40°C to about 80°C, more preferably about 80°C. and a drying time of about 4 hours to about 8 hours, more preferably about 6 hours at 40°C.

The dried granules are sized by passage through a sieve preferably of 12-40 mesh. A 20 mesh sieve is most preferred.

The sized granules are mixed with a lubricant prior to being pressed into tablets. The most preferred lubricant is magnesium stearate. The granules and lubricant are preferably mixed to homogeneity. In certain embodiments, mixing is conducted for about 1 to about 60 minutes, and preferably about 10 minutes.

The mixture of granules and lubricant is compressed into tablets, preferably using a rotary tablet machine. In certain embodiments, about 5,000 to about 300,000 tablets are produced per hour with a hardness of about 20N to about 300N. The shape of the tablets is not particularly limited. In certain embodiments, the tablets are flat and plain and have a diameter of about 8.0 mm.

To investigate the influence of the particle sizes of alfuzosin hydrochloride and additives, the particle size distribution was determined by sieve analysis. Differences in particle size distribution exist between alfuzosin hydrochloride, HPMC and lactose monohydrate. The mixing step prior to granulation and the wet granulation process do not compensate for these differences. This could lead to separation in the hopper or the feed frame of the tablet press.

Therefore, it is preferred to include a grinding step in the process of producing the composition of the invention to reduce the particle size of the alfuzosin hydrochloride. The coarse material is preferably ground into small particles of less than about 0.25 mm diameter prior to being combined with the other ingredients.

The preferred particle size specification for alfuzosin hydrochloride is as follows:

|  | Unit | Lower Limit | Upper Limit | value(n=3) |
|---|---|---|---|---|
| Particle size < 0.25 mm | % | 85 | 95 | 90 |
| Particle size < 0.5 mm | % | 90 | 100 | 96 |
| Particle size < 1.0 mm | % | 100 | 100 | 100 |

The invention will be illustrated in more detail with reference to the following Examples, but it should be understood that the present invention is not deemed to be limited thereto.

EXAMPLES

The objective of the Examples is to demonstrate that a composition of the invention achieves sustained-release of alfuzosin hydrochloride, resulting in alfuzosin plasma concentration profiles corresponding to those of the product marketed outside the U.S. under the trade name XATRAL LP 10 mg ("the reference formulation"). The reference formulation is a triple-layer tablet with alfuzosin hydrochloride provided in the middle layer. The upper and lower layers contain the swelling polymer HPMC.

A dissolution test with adequate discriminatory power was developed to measure the dissolution profiles of the samples tested. Samples were tested in a basket apparatus, as described in the Ph. Eur. and the USP, using a rotation speed of 100 rpm. The compositions of the buffers used and the test parameters were as follows:

0.01N HCl solution pH 2.0
Medium: 1000 ml 0.01N HCl (pH 2.0)
Basket method (Basket 40 mesh cloth (USP))
Temperature: 37 ± 0.5°C
Time intervals of measurement: 30 min, 1h for up to 12 h
Wavelength: 244 nm
Path length of cuvette: 10 mm
Rotation speed: 100 rpm
Filter: 25 mm D Whatman glass micro fiber filter, type GF/D or equivalent
Apparatus: Sotax AT 7-On-Line

The amount of dissolved alfuzosin hydrochloride is determined as follows:

Calculation of extinction coefficient:

$$E^1_{1\,std} = \frac{A_{std} \cdot 10000 \cdot 1000}{C_{std} \cdot L_{std} \cdot P_{std}}$$

= Extinction coefficient of a 1% (m/V) solution of standard in a 1 cm cell
$A_{sld}$ = Absorbance of the standard solution
C = Concentration of the standard solution [mg/l]
L = Path length of standard cuvette [mm]
B = Purity of the standard substance [%]

$$C_{corsamp} = \frac{A_{samp} \cdot 10 \cdot 1000 \cdot M \cdot 100}{E^1_{1std} \cdot L_{samp} \cdot 100 \cdot P_{samp}}$$

Concentration calculation:

C = Weight corrected concentration of the sample solution [mg/bath volume

A = Absorbance of the sample solution

M = Media volume [ml]

$E^1_{1\,std}$ = Extinction coefficient of a 1% (m/V) solution of standard in a 1 cm cell

$L_{sump}$ = Path length of sample cuvette [mm]

$P_{sump}$ = Purity of the sample (drug substance) [%]

Calculation of percentage dissolution:

$$\% \, Dissolution = \frac{C_{corsump} \cdot 100}{D}$$

$C_{consigne}$ - Weight corrected concentration of the sample solution [mg/bach volume]

D = Theuretical amount of drug substance/ tablet

Tablets of the invention were prepared to contain 10 mg of alfuzosin hydrochloride in a 300 mg tablet. Spray dried lactose monohydrate was employed as a water-soluble filler to maintain the weight of the tablet. Due to its water solubility, lactose monohydrate is an effective release enhancer of alfuzosin hydrochloride in the matrix system.

EXAMPLES 1-12

First attempts were made to manufacture the tablets by direct compression after blending the active substance with suitable excipients. For this purpose 10, 30, 50 and 70% HPMC with various viscosity grades (4000 cp, 15000cp, 100000cp) were mixed with alfuzosin hydrochloride and lactose monohydrate as a channel forming agent. The excipients were wet granulated in a high shear mixer. After drying, the granules were collected and delumped in an oscillating granulator. Finally magnesium stearate was mixed with the granules in a cube mixer. The tablets were compressed at a hardness of 100 N and 140 N. The compositions of the produced batches are summarized in the following tables.

Table 2. Examples 1-4 containing HPMC 100000cP

| INGREDIENT | EXAMPLE | | | |
|---|---|---|---|---|
| | 1 | 2 | 3 | 4 |
| | (mg/tab) | (mg/tab) | (mg/tab) | (mg/tab) |
| Alfuzosin HCl | 10.28 | 10.28 | 10.28 | 10.28 |
| Lactose monohydrate Pharmatose DCL 11 | 64.72 | 124.72 | 184.72 | 244.72 |
| Hypromellose Metolose 90SH100000 | 210 | 150 | 90 | 30 |
| Povidone K25 | 12 | 12 | 12 | 12 |
| Magnesium stearate | 3 | 3 | 3 | 3 |
| Total | 300 | 300 | 300 | 300 |

Table 3. Examples 5-8 containing HPMC 15000cP

| INGREDIENT | EXAMPLE | | | |
|---|---|---|---|---|
| | 5 | 6 | 7 | 8 |
| | (mg/tab) | (mg/tab) | (mg/tab) | (mg/tab) |
| Alfuzosin HCl | 10.28 | 10.28 | 10.28 | 10.28 |
| Lactose monohydrate Pharmatose DCL 11 | 64.72 | 124.72 | 184.72 | 244.72 |
| Hypromellose Metolose 90SH 150000 | 210 | 150 | 90 | 30 |
| Povidone K25 | 12 | 12 | 12 | 12 |
| Magnesium stearate | 3 | 3 | 3 | 3 |
| Total | 300 | 300 | 300 | 300 |

Table 4. Examples 9-12 containing HPMC 4000cP

| INGREDIENT | EXAMPLE | | | |
|---|---|---|---|---|
| | 9 | 10 | 11 | 12 |
| | (mg/tab) | (mg/tab) | (mg/tab) | (mg/tab) |
| Alfuzosin HCl | 10.28 | 10.28 | 10.28 | 10.28 |
| Lactose monohydrate Pharmatose DCL 11 | 64.72 | 124.72 | 184.72 | 244.72 |
| Hypromellose Metolose 90SH 4000 | 210 | 150 | 90 | 30 |
| Povidone K25 | 12 | 12 | 12 | 12 |
| Magnesium stearate | 3 | 3 | 3 | 3 |
| Total | 300 | 300 | 300 | 300 |

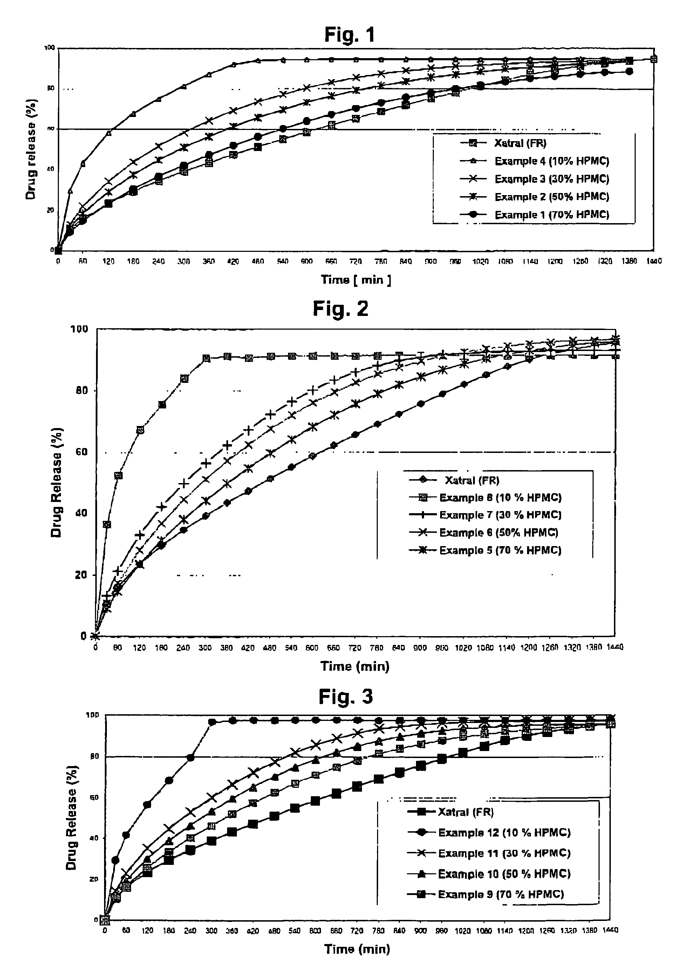

Dissolution profiles of Examples 1-12 were recorded and the results are shown in Figs. 1-3. The dissolution profiles clearly show that the matrix formulation with a higher amount of HPMC shows a release profile closer to the reference formulation. Prior to the direct compression trial the flowability of those batches has been evaluated. The values obtained demonstrated that the higher the HPMC concentration, the lower the flowability. The formula containing 70% of HPMC was close to the reference formulation but showed less than ideal flow characteristics.

EXAMPLES 13-15

Tablets were manufactured by a wet granulation process comprising the following steps:

1) Alfuzosin hydrochloride and lactose monohydrate are weighed, taking into account the assay and water content of Alfuzosin hydrochloride. The calculation of quantities to be weighed is carried out by the following formulas:

$$Q_x = \frac{S_A \cdot 100 \cdot 100}{A \cdot W'} \ [\text{kg}]$$

$$Q_y = S_L - Q_x \ [\text{kg}]$$

Q Quantity of alfuzosin hydrochloride
$Q_y$ Quantity of lactose monohydrate
$S_A$ Standard quantity of alfuzusin hydrochloride
$S_L$ Sum of standard quantities of alfuzosin hydrochloride and lactose monohydrate

W' (100 - Water content) [%]

A Assay on anhydrous basis [%]

Alfuzosin hydrochloride, lactose monohydrate (Pharmatose DCL 11) and hypromcllose (Metolose 60 SH 100000) are mixed in a high shear mixer.

2) Povidone (K25) is added to a suitable amount of water and dissolved under continuous stirring to provide a lump free binder solution.

3) The binder solution is added to the dry mix (of step 1) for wet granulation.

4) The granules are dried at ≥80 °C until the desired loss on drying-value is achieved.

5) The dried granules are sifted through a 20# sieve.

6) The granulate and magnesium stearate are filled into a container mixer. The compounds are mixed for 10 minutes.

7) The mixture obtained from step 6 is compressed to tablets on a rotary tablet machine.

Studies were then conducted to establish the amount (%) and viscosity grade of the gel forming HPMC in a prolonged release formulation with a release profile similar to the reference product. For that reason 70, 80 and 90% HPMC, based on the tablet weight, were employed in the test formulations. For maintaining the weight of the tablet, spray dried lactose monohydrate was employed. The sample formulations of each batch are summarized in Table 5. For each HPMC grade, one test formulation was manufactured:

Table 5. Examples 13-15 Formulations containing HPMC 100000 cP

| INGREDIENT | EXAMPLE | | |
|---|---|---|---|
| | 13 | 14 | 15 |
| | (mg/tab) | (mg/tab) | (mg/tab) |
| Alfuzosin HCl | 10.2 | 10.2 | 10.2 |
| Lactose monohydrate Pharmatose DCL 11 | 64.8 | 34.8 | 4.8 |
| Hypromellose Metolose 90SH 100000 | 210 | 240 | 270 |
| Povidone K25 | 12 | 12 | 12 |
| Magnesium stearate | 3 | 3 | 3 |
| Total | 300 | 300 | 300 |

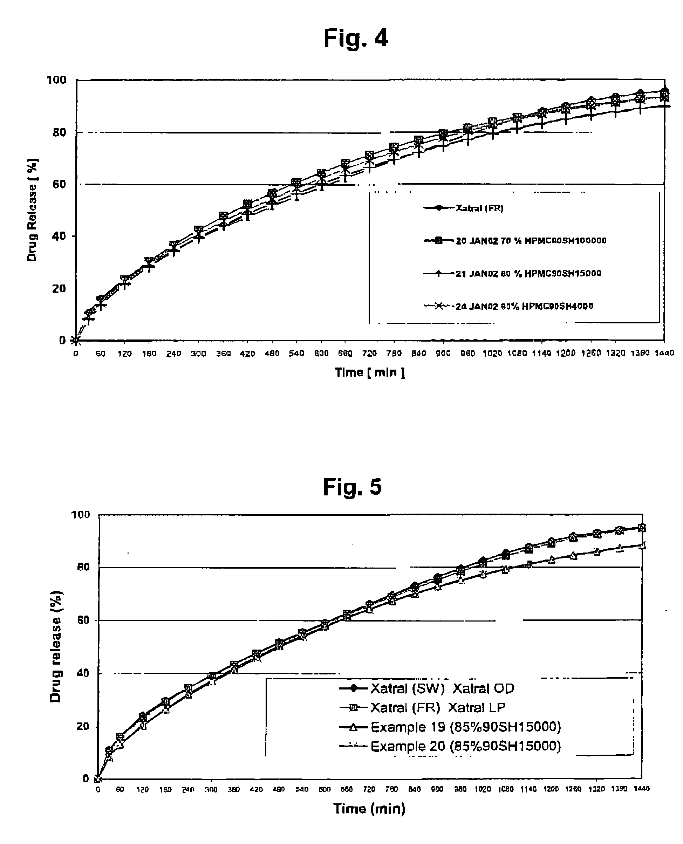

Dissolution profiles of Examples 13-15 were recorded and the results are shown in Fig. 4 relative to the reference formulation. All dissolution profiles were comparable.

EXAMPLES 16-18

It was decided to further optimize the formulation variant with the HPMC having a viscosity grade of 15000cP, as this formulation also shows good manufacturing properties. For that purpose, batches containing 85% HPMC instead of 80% and 24 mg instead of 12 mg of povidone per tablet were manufactured and different granulation liquids (ethanol, purified water) were tested, as shown in Table 6.

Table 6. Examples 16-18 Formulations containing HPMC 15000 cP

| Ingredients (mg/tab) | Example 16 | Example 17 | Example 18 |
|---|---|---|---|
| Alfuzosin Hydrochloride | 10 | 10 | 10 |
| Hypromellose 15000cP | 255 | 255 | 255 |
| Lactose H2O | 8 | 8 | 8 |
| Total | 300 | 300 | 300 |
| Binder solution | | | |
| Polyvinylpyrrolidone | 24 | 24 | 24 |
| Ethanol | | ✔ | ✔ |
| Purified water | ✔ | ✔ | |

Table continued

| Ingredients (mg/tab) | Example 16 | Example 17 | Example 18 |
|---|---|---|---|
| Lubrication | | | |
| Magnesium stearate | 3 | 3 | 3 |
| Granules | | | |
| Loss on drying % | 4.09 | 3.96 | 3.07 |
| Bulk density g/cm$^3$ | 0.399 | 0.425 | 0.276 |
| Tapped density g/cm$^3$ | 0.503 | 0.520 | 0.368 |
| Hausner ratio | 1.26 | 1.22 | 1.33 |
| Flowability | fair | good | poor |
| Tablets | | | |
| Hardness N | 110 | 100 | --- |
| Friability % | 0.17 | 0.16 | --- |
| Weight variation RSD % | 1.81 | 2.27 | --- |

To prepare the tablets, the alfuzosin hydrochloride was mixed with HPMC, lactose monohydrate and povidone. The wet granulation process was performed as described in Examples 13-15 with the solvents modified as noted in Table 6 and the povidone added to the dry mix.

Example 18 showed a decrease in bulk/ tapped density due to the ethanolic granulation. The tablet could not be pressed. Granules of Example 17 showed the best flowability. This was confirmed by results of the weight variation (RSD of 1.8% for Example 16 versus 2.3% for Example 17. Therefore, the aqueous granulation is preferred to the alcoholic or water/alcohol granulation liquid.

EXAMPLES 19-20

Two additional batches containing 85% HPMC were prepared using the wet granulation process of Examples 13-15 to assess the reproducibility of the formulation. The formulations of the two batches were the same, as shown in Table 7.

Table 7. Examples 19-20 Formulations

| Ingredient | Amount per dosage form (mg) |
|---|---|
| Alfuzosin hydrochloride | 10.00 |
| Lactose monohydrate | 8.00 |
| Hypromellose | 255.00 |
| Povidone K25 | 24.00 |
| Purified water[+] | 24.00 |
| Magnesium stearate | 3.00 |
| Total | 300.00 |

[+]Not contained in the finished product

Dissolution profiles were recorded to compare the above mentioned formulation with two batches of the reference product. The tablets are dissolved in 0.01 N HCl solution (pH 2.0). The resulting dissolution profiles are shown in Fig. 5. The dissolution profiles of the batches containing 85% HPMC 15000 cp and 8% povidone are comparable with the release profiles of the reference formulations XATRAL OD and XATRAL LP 10 mg.

EXAMPLE 21

To investigate the dependency of the alfuzosin hydrochloride release on tablet hardness, dissolution profiles were recorded in 0.01 HCl solution at pH 2.0. For this purpose, tablets containing 85% HPMC and having a tablet-hardness between 135 N and 225 N were manufactured. The resulting dissolution profiles (not shown) demonstrate that the release of the drug substance is independent of the hardness within the tested range.

EXAMPLES 22-32

The propensity of compositions to float was studied in Examples 22-32. Sec Table 8 below. Tablets of Examples 26, 30 and 31 were prepared according to Examples 1-12. Tablets of Examples 22-25, 27-29 and 32 were prepared according to Examples 13-15.

Table 8. Examples 22-32.

| Ingredients (mg/Tablet) | Example No. | | | | | | | | | | |
|---|---|---|---|---|---|---|---|---|---|---|---|
| | 22 | 23 | 24 | 25 | 26 | 27* | 28* | 29 | 30* | 31* | 32* |
| alfuzosin anhydrate | 10 | 10 | 10 | 10 | 10 | 10 | 10 | 10 | 10 | 10 | 10 |
| lactose | 8 | 8 | 8 | 8 | 8 | | | | | 120 | |
| cornstarch | | | | | | 8 | | | | | |
| Sorbitol | | | | | | | 8 | | | | |
| cellulose microcristalline | | | | | | | | 8 | | | 8 |
| HPMC Metolose 90 SH 15 000 | 255 | 255 | 255 | 255 | 255 | 255 | 255 | 255 | 390 | | |
| HPMC Metolose 90 SH 4000 | | | | | | | | | | 270 | 255 |
| Plasdone K 25 | 24 | 24 | 24 | 24 | 24 | 24 | 24 | 24 | | | 24 |
| magnesium stearate | 3 | 3 | 3 | 3 | 3 | 3 | 3 | 3 | | | 3 |
| water purified | 10 | | | | | 36 | 36 | 36 | | | 36 |
| ethanol | | 117 | | 166 | | | | | | | |
| isopropanol | | | 217 | | | | | | | | |
| | | | | | | | | | | | |
| **Preparation Method** | | | | | | | | | | | |
| Dry granulation | -- | -- | -- | -- | yes | -- | -- | -- | yes | yes | -- |
| Wet granulation (aqueous) | yes | -- | -- | -- | -- | yes | yes | yes | -- | -- | yes |
| Wet granulation (organic) | -- | yes | yes | yes | -- | -- | -- | -- | -- | -- | -- |
| | | | | | | | | | | | |
| **Result** | | | | | | | | | | | |
| Flotation | no | no | no | no | no | yes | yes | no | yes | yes | yes |
| * comparative example | | | | | | | | | | | |

Examples 22-26 show that the preferred compositions of the invention do not float regardless of which granulation method/liquid is used to prepare them.
Examples 27-28 are comparative examples showing that the substitution of cornstarch or sorbitol for the lactose in the compositions of the invention results in flotation.
Example 29 shows that the substitution of microcrystalline cellulose for lactose does not result in flotation.
Examples 30-31 are comparative examples prepared in accordance with the teachings of Bordes et al. discussed above, which resulted in floating tablets.
Example 32 is a comparative example, in which the substitution of Metolose SH 90 4000 for Metolose SH 90 15000 results in undesired flotation compared to Example 29.

EXAMPLES 33-38

The effect of tablet formulation on the alfuzosin polymorph present in the formulation was studied in Examples 33-38. See Table 9 below. The granulation methods of Examples 22-32 were used to prepare the samples.

Table 9. Examples 33-38.

| Ingredients (mg/Tablet) | Example No. | | | | | |
|---|---|---|---|---|---|---|
| | 33 | 34 | 35 | 36 | 37 | 38* |
| alfuzosin anhydrate | 10 | 10 | 10 | 10 | 10 | 10 |
| lactose | 8 | 8 | 8 | 8 | 8 | |
| cornstarch | | | | | | 8 |
| Sorbitol | | | | | | |
| cellulose microcristalline | | | | | | |
| HPMC Metolose 90 SH 15 000 | 255 | 255 | 255 | 255 | 255 | 255 |
| HPMC Metolose 90 SH 4000 | | | | | | |
| Plasdone K 25 | 24 | 24 | 24 | 24 | 24 | 24 |
| magnesium stearate | 3 | 3 | 3 | 3 | 3 | 3 |
| water purified | 10 | | | | | 20 |
| ethanol | | 117 | | 166 | | |
| isopropanol | | | 217 | | | |
| | | | | | | |
| **Preparation Method** | | | | | | |
| Dry granulation | -- | -- | -- | -- | yes | -- |
| Wet granulation (aqueous) | yes -- | -- | -- | -- | -- | yes |
| Wet granulation (organic) | -- | yes | yes | yes | -- | -- |
| | | | | | | |
| **Result** | | | | | | |
| Anhydrate Stabilization | yes | yes | yes | yes | yes | no |
| * comparative example | | | | | | |

Examples 33-37 show that the preferred formulations stabilize the alfuzosin anhydrate regardless of the manufacturing method employed.
Example 38 is a comparative example showing that the substitution of cornstarch for lactose transformed the anhydrate polymorph to the dihydrate polymorph.
While the invention has been described in detail and with reference to specific examples thereof, it will be apparent to one skilled in the art that various changes and modifications can be made therein without departing from the spirit and scope thereof.

**Claims**

1.   A composition comprising:

a polymeric matrix; and
alfuzosin in the polymeric matrix,

wherein: (a) the composition is monolithic in form, (b) the composition does not float in gastric fluids, and (c) the polymeric matrix is adapted to release 13-33% of the alfuzosin within 2 hours of administration, 40-60% of the alfuzosin within 7 hours of administration and greater than 80% of the alfuzosin within 20 hours of administration.

2. The composition of claim 1, wherein the alfuzosin is an anhydrous salt.

3. The composition of claim 1, wherein the alfuzosin consists essentially of alfuzosin hydrochloride anhydrate.

4. The composition of claim 1, wherein the composition is provided as a tablet free of layers.

5. The composition of any of claims 1 to 4, wherein the alfuzosin is homogeneously distributed throughout the composition.

6. The composition of any of claims 1 to 5, wherein the polymeric matrix is homogeneously distributed throughout the composition.

7. The composition of any of claims 1 to 6, wherein the polymeric matrix predominantly comprises a hydrophilic polymer adapted to gel or swell upon contact with gastrointestinal fluids.

8. The composition of claim 7, wherein the hydrophilic polymer is hydroxypropylmethylcellulose.

9. The composition of claim 8, further comprising lactose monohydrate, polyvinylpyrrolidone and magnesium stearate.

10. The composition of any of claims 1 to 9, comprising 1-30 mg alfuzosin hydrochloride anhydrate, 2-100 mg lactose monohydrate, 20-800 mg hydroxypropylmethylcellulose, 2-100 mg polyvinylpyrrolidone and 0.1-25 mg magnesium stearate.

11. The composition of any of claims 1 to 10, comprising about 10 mg alfuzosin hydrochloride anhydrate, about 7.8 mg lactose monohydrate, about 255 mg hydroxypropylmethylcellulose, about 24mg polyvinylpyrrolidone and about 3.0 mg magnesium stearate.

12. The composition of any of claims 1 to 11, wherein the composition is adapted to induce a peak plasma concentration of alfuzosin about 6 hours to about 9 hours after oral administration.

13. The composition of claim 12, wherein an elimination half-life is about 9 hours after oral administration.

14. The composition of any of claims 1 to 12, produced by a dry, aqueous or organic granulation process.

15. A composition comprising:

   a polymeric matrix; and
   alfuzosin in the polymeric matrix,

   wherein: (a) the composition is monolithic in form, (b) a 300 mg tablet of the composition having a hardness of 100 N sinks within one minute of being placed in one liter of a 0.01 N HCl solution in a USP Type II apparatus with a paddle speed of 100 rpm and a temperature of $37\pm2°C$, and remains below the surface for at least 20 minutes, and (c) the polymeric matrix is adapted to release 13-33% of the alfuzosin within 2 hours of administration, 40-60% of the alfuzosin within 7 hours of administration and greater than 80% of the alfuzosin within 20 hours of administration.

16. The composition of claim 15, wherein the alfuzosin is an anhydrous salt.

17. A unit dosage form comprising a heterogeneous mixture of alfuzosin hydrochloride anhydrate, lactose monohydrate, hydroxy propylmethylcellulose, polyvinylpyrrolidone and magnesium stearate, wherein the heterogeneous mixture is heterogeneously distributed throughout the unit dosage form, and the unit dosage form does not float in gastric fluids.

18. The unit dosage form of claim 17, comprising 1-30 m g alfuzosin hydrochloride anhydrate, 2-100 mg lactose monohydrate, 20-800 mg hydroxypropylmethylcellulose, 2-100 mg polyvinylpyrrolidone and 0.1-25 mg magnesium stearate.

**19.** The unit dosage form of claim 17 or 18, comprising about 10 mg alfuzosin hydrochloride anhydrate, about 7.8 mg lactose monohydrate, about 255 mg hydroxypropylmethylcellulose, about 24mg polyvinylpyrrolidone and about 3.0 mg magnesium stearate.

**20.** The unit dosage form of any of claims 17, 18 or 19 adapted to release 13-33% of the alfuzosin within 2 hours of administration, 40-60% of the alfuzosin within 7 hours of administration and greater than 30% of the alfuzosin within 20 hours of administration.

**21.** The unit dosage form of any of claims 17 to 20, wherein the unit dosage form is a monolithic tablet.

**22.** The unit dosage form of any of claims 17 to 21, having a pharmaceutically inactive external coating.

**23.** The unit dosage form of any of claims 17 to 22, being substantially free of polymorphs of alfuzosin other than alfuzosin hydrochloride anhydrate.

**24.** A process for preparing the composition of claim 1, said process comprising:

> mixing a hydrophilic polymer and alfuzosin to provide a blend;
> granulating the blend to provide granules;
> drying the granules on a dryer to provide dried granules;
> sizing the dried granules to provide sized granules;
> mixing the sized granules with a lubricant to obtain a mixture; and
> compressing the mixture to obtain a tablet.

**25.** The process of claim 24, wherein the hydrophilic polymer is hydroxypropylmethylcellulose and the alfuzosin is alfuzosin hydrochloride anhydrate.

**26.** The process of claim 24 or 25, wherein the granulating comprises dry granulating the blend or adding a granulation liquid to the blend and wet granulating the blend.

**27.** The process of claim 26, wherein the blend comprises hydroxypropylmethylcellulose, alfuzosin hydrochloride anhydrate, polyvinylpyrrolidone and lactose monohydrate, the granulating comprises dry granulating or wet granulating with a granulation liquid comprising at least one of alcohol and water and optionally polyvinylpyrrolidone, and the lubricant comprises magnesium stearate.

**28.** The process of claim 27, wherein the tablet contains 1-30 mg alfuzosin hydrochloride anhydrate, 2-100 mg lactose monohydratc, 20-800 mg hydroxypropylmethylcellulose, 2-100 mg polyvinylpyrrolidone and 0.1-25 mg magnesium stearate.

**29.** The process of claim 27 or 28, wherein the tablet contains about 10 mg alfuzosin hydrochloride anhydrate, about 7.8 mg lactose monohydrate, about 255 mg hydroxypropylmethylcellulose, about 24mg polyvinylpyrrolidone and about 3.0 mg magnesium stearate.

**30.** Use of the composition of claim 1 for the manufacture of a medicament for the treatment of benign prostatic hyperplasia.

**Fig. 1**

**Fig. 2**

**Fig. 3**

# Fig. 4

# Fig. 5

**European Patent Office**

**EUROPEAN SEARCH REPORT**

Application Number

EP 05 01 1338

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 2004/037228 A (RANBAXY LABORATORIES LIMITED; VISWANATHAN, NARAYANAN, BADRI; RAMAKRISH) 6 May 2004 (2004-05-06) * page 3, line 21 - line 32 * * page 6, line 14 - line 20 * * examples * * claims * | 1-10, 12-18, 20-28,30 | A61K9/22 A61K31/517 |
| A | FR 2 820 319 A (ELLIPSE PHARMACEUTICALS) 9 August 2002 (2002-08-09) * page 3, line 4 - line 29 * * examples * * claims * | 1-30 | |
| D,A | US 6 149 940 A (MAGGI ET AL) 21 November 2000 (2000-11-21) * column 2, line 1 - line 36 * * claims; examples * | 1-30 | |
| A | US 5 589 190 A (ANDRIEU ET AL) 31 December 1996 (1996-12-31) * claims; examples * | 1-30 | TECHNICAL FIELDS SEARCHED (Int.Cl.7) A61K |

The present search report has been drawn up for all claims

| Place of search | Date of completion of the search | Examiner |
|---|---|---|
| The Hague | 25 October 2005 | Epskamp, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 01 1338

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2005

| Patent document cited in search report | | Publication date | Patent family member(s) | | Publication date |
|---|---|---|---|---|---|
| WO 2004037228 | A | 06-05-2004 | AU | 2003267732 A1 | 04-05-2004 |
| | | | AU | 2003278407 A1 | 13-05-2004 |
| | | | BR | 0315569 A | 30-08-2005 |
| | | | EP | 1558218 A1 | 03-08-2005 |
| | | | WO | 2004032905 A1 | 22-04-2004 |
| FR 2820319 | A | 09-08-2002 | CA | 2437630 A1 | 15-08-2002 |
| | | | EP | 1368002 A2 | 10-12-2003 |
| | | | WO | 02062321 A2 | 15-08-2002 |
| | | | US | 2004115259 A1 | 17-06-2004 |
| US 6149940 | A | 21-11-2000 | AT | 200864 T | 15-05-2001 |
| | | | AU | 724490 B2 | 21-09-2000 |
| | | | AU | 4020197 A | 19-03-1998 |
| | | | BG | 63768 B1 | 29-12-2002 |
| | | | BG | 103138 A | 30-09-1999 |
| | | | BR | 9713237 A | 04-04-2000 |
| | | | CA | 2264250 A1 | 05-03-1998 |
| | | | CN | 1228700 A | 15-09-1999 |
| | | | CN | 1543954 A | 10-11-2004 |
| | | | CY | 2284 A | 04-07-2003 |
| | | | CZ | 9900655 A3 | 16-06-1999 |
| | | | DE | 69704712 D1 | 07-06-2001 |
| | | | DE | 69704712 T2 | 29-11-2001 |
| | | | DK | 938318 T3 | 20-08-2001 |
| | | | EE | 9900090 A | 15-10-1999 |
| | | | EP | 0938318 A1 | 01-09-1999 |
| | | | ES | 2159400 T3 | 01-10-2001 |
| | | | WO | 9808515 A1 | 05-03-1998 |
| | | | GR | 3036291 T3 | 31-10-2001 |
| | | | HK | 1020875 A1 | 23-11-2001 |
| | | | IL | 128146 A | 11-01-2001 |
| | | | JP | 2000514462 T | 31-10-2000 |
| | | | KR | 2000035929 A | 26-06-2000 |
| | | | NO | 990944 A | 27-04-1999 |
| | | | NZ | 334018 A | 29-06-1999 |
| | | | OA | 10983 A | 04-03-2003 |
| | | | PL | 331862 A1 | 16-08-1999 |
| | | | PT | 938318 T | 30-10-2001 |
| | | | RU | 2183459 C2 | 20-06-2002 |
| | | | SK | 27099 A3 | 10-12-1999 |
| | | | TR | 9900289 T2 | 21-12-2000 |
| | | | TW | 522023 B | 01-03-2003 |
| US 5589190 | A | 31-12-1996 | AT | 203672 T | 15-08-2001 |
| | | | AU | 681236 B2 | 21-08-1997 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 05 01 1338

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2005

| Patent document cited in search report | Publication date | Patent family member(s) | Publication date |
|---|---|---|---|
| US 5589190 A | | AU 1495595 A | 28-09-1995 |
| | | CA 2144917 A1 | 22-09-1995 |
| | | CN 1116524 A | 14-02-1996 |
| | | CZ 9500704 A3 | 18-10-1995 |
| | | DE 69521924 D1 | 06-09-2001 |
| | | DE 69521924 T2 | 11-04-2002 |
| | | EP 0673650 A1 | 27-09-1995 |
| | | FI 951300 A | 22-09-1995 |
| | | FR 2717388 A1 | 22-09-1995 |
| | | HU 72635 A2 | 28-05-1996 |
| | | IL 113053 A | 22-12-1999 |
| | | JP 7258085 A | 09-10-1995 |
| | | NO 951055 A | 22-09-1995 |
| | | NZ 270748 A | 21-12-1995 |
| | | PL 307773 A1 | 02-10-1995 |
| | | SK 36595 A3 | 08-11-1995 |
| | | ZA 9502290 A | 24-01-1996 |

EPO FORM P0459

For more details about this annex : see Official Journal of the European Patent Office, No. 12/82